# EUROPEAN PATENT SPECIFICATION

(11) **EP 1 107 000 B1**
(45) Date of publication and mention of the grant of the patent: **02.11.2005**
(21) Application number: 00204289.3
(22) Date of filing: 01.12.2000
(51) Int. Cl.: G01N 33/02, G01N 3/40

(54) **Method and device for determining the hardness/elasticity and/or other properties of vegetables or fruit**
Verfahren und Vorrichtung zur Bestimmung der Härte/Elastizität und/oder anderer Eigenschaften von Gemüsen oder Obst
Méthode et appareil pour déterminer la dureté/élasticité et/ou autres caractéristiques de légumes ou fruits

(30) Priority: 01.12.1999 NL 1013720; 16.08.2000 NL 1015949
(43) Date of publication of application: 13.06.2001
(73) Proprietor: De Greef's Wagen- Carrosserie- en Machinebouw B.V., NL-4196 AM Tricht (NL)
(72) Inventor: de Greef, Jacob Hendrik, 4181 BM Waardenburg (NL)
(74) Representative: Land, Addick Adrianus Gosling

(56) References cited:
- WO-A-98/40737
- WO-A-98/52037
- FR-A- 2 702 048
- US-A- 4 061 020
- US-A- 5 315 879
- US-A- 5 806 686
- US-A- 5 811 680

## Description

Vegetables and fruit such as apples, pears, tomatoes, citrus fruits and the like are selected in current practice on the basis of a large number of parameters, such as size, weight, colour and the like. Because of increasing demands of consumers, there is now a trend toward also sorting fruits and vegetables according to hardness.

It is the current practice at auctions for an inspector to determine the hardness of a fruit in usually destructive manner by means of penetration with an impactor through about 2 cm. This hardness can have different values at different locations on the fruit. In the classification and selection of fruits there is therefore a need to determine the hardness of a fruit on the surface in non-destructive manner, for instance by means of non-destructive impact (or hardness) measurements.

US-A-5,315,879 discloses a method and apparatus for determining the ripeness of fruits by measuring the firmness at several locations on the surface of the fruit. Fruits are continuously transported by a conveyor system formed by an endless chain of waisted cylinders that rotate in the same direction as their drive direction, thereby rotating the fruits. The device comprises a plurality of shoes supported by movable arms, each shoe supporting at least one sensor for measuring the firmness of the fruit.

In the international patent application WO9852037 an impactor in a bellows is carried above one fruit or vegetable at a time, whereafter the local hardness derived from the elasticity is determined from the impact measured with a piezo-electric element.

This known method and device is however not suitable for performing measurements on fruits being supplied on a conveyor at a high speed, since the bellows must be brought close to the fruit each time. The hardness (or elasticity) of the fruit is furthermore determined at only one position, which provides an unreliable indication of the general hardness (and/or ripeness) of that fruit.

The present invention provides a method and a device for determining the hardness of a vegetable or fruit comprising the features defined in the appended claims. In this device, the vegetable or fruit is rotated, wherein an impactor element is carried close to the surface of a fruit or vegetable, wherein during rotation of the fruit an impactor element is brought into contact with the fruit or the vegetable a number of times, wherein the impact of the respective impactor element is measured in order to determine the hardness of the vegetable or fruit over at least a portion of the surface thereof.

The impactor element co-displaces with the fruit above the conveyor in order to enable a large number of measurements over the surface thereof while the fruit is supplied by the supply conveyor.

In another preferred embodiment of the method and device according to the present invention, it is likewise conceivable for a number of impactor elements to be in successive stationary disposition while the fruits are transported rotatingly thereunder (or over) on a conveyor. It is of course of equal importance here that the impactor elements can properly follow the contours of successive fruits or vegetables of perhaps differing diameter, for which purpose it is important that the impactor elements are freely movable in vertical direction.

The device for determining the hardness of a vegetable or fruit basically comprises:
- a supply conveyor for supplying the vegetables or fruits,
- rotation means for causing the vegetables or fruits to rotate on the supply conveyor; and
- one or more hardness measuring members for measuring the hardness of the vegetables or fruits during rotation thereof, wherein the hardness measuring members are arranged above the supply conveyor.

In a further preferred embodiment of the device according to the present invention the hardness measuring members are arranged pivotally relative to each other or movable in height independently of each other in order to perform reliable hardness measurements on successive vegetables or fruits of different size.

In a first preferred embodiment the impactor element is lifted using a solenoid and then released, whereafter the impact thereof is measured using a piezoelectric transducer; in other embodiments mechanical lifting of the weight on the impactor element can also take place, wherein the impact is likewise determined by means of a piezo-electric transducer or with other means.

In a further preferred embodiment the hardness or impact transducer is arranged adjacently to a wheel or disc which rolls along the surface of a fruit and which, irrespective of the diameter thereof, prevents damage to this fruit. In the embodiment wherein the transducer co-displaces with the fruit, the hardness can be determined at any desired location along the surface, which increases the flexibility of the number of measurements and the speed of the conveyor.

The method and device according to the present invention can also be applied for other measurement principles, such as to determine the spectrum of the light reflected by the surface of a vegetable or fruit, the spectrum of the light passing through the object, the roughness of the skin of a vegetable or fruit, the analysis of the gases given off by the object and the like.

The present invention therefore also provides a method for determining a property of a vegetable or fruit, wherein the vegetable or fruit is rotated, wherein during rotation of the fruit the transducer is brought a number of times into the vicinity of the fruit or the vegetable, wherein the property is measured, in order to determine the property of a vegetable or fruit over at least a portion of the surface thereof.

The present invention further provides a device for performing such a method.

In order to prevent an (elongate) fruit which is supplied on a brush roller being forced laterally out of the conveyor path due to contact with a wheel or transducer, in accordance with another aspect the present invention provides a brush roller with one or more, preferably two, rings of slip-resistant material such as rubber. In a further preferred embodiment a sensor element or contact element is arranged between two diabolo-shaped elements in order to position the fruits properly and to obtain a reliable and accurate measuring result.

Further advantages, features and details of the present invention will be elucidated on the basis of the following description with reference to the annexed drawings, in which:
figure 1 shows a schematic view in perspective of a preferred embodiment of the device and method according to the present invention;
figure 2 shows a perspective view of detail II of figure 1;
figure 3 is a front view in perspective of detail III of figure 2;
figures 4A - 4E are schematic side views of respective alternative embodiments of an impactor element to be applied in the embodiment according to figure 1;
figure 5 shows a schematic side view of a further preferred embodiment of a device and method according to the present invention;
figure 6 shows a view of a further preferred embodiment of the device as according to figures 1, 2 and 3;
figure 7 shows a schematic view of a further preferred embodiment of a method and device according to the present invention;
figures 8, 9 and 10 are respective schematic views of further preferred embodiments of a method and device according to the present invention;
figure 11 is a view of a brush roller with two slip-resistant rings;
figures 12 and 13 show further preferred embodiments of a part of a device according to the present invention; and
figure 14 is a schematic side view of another preferred embodiment of a device according to the present invention.

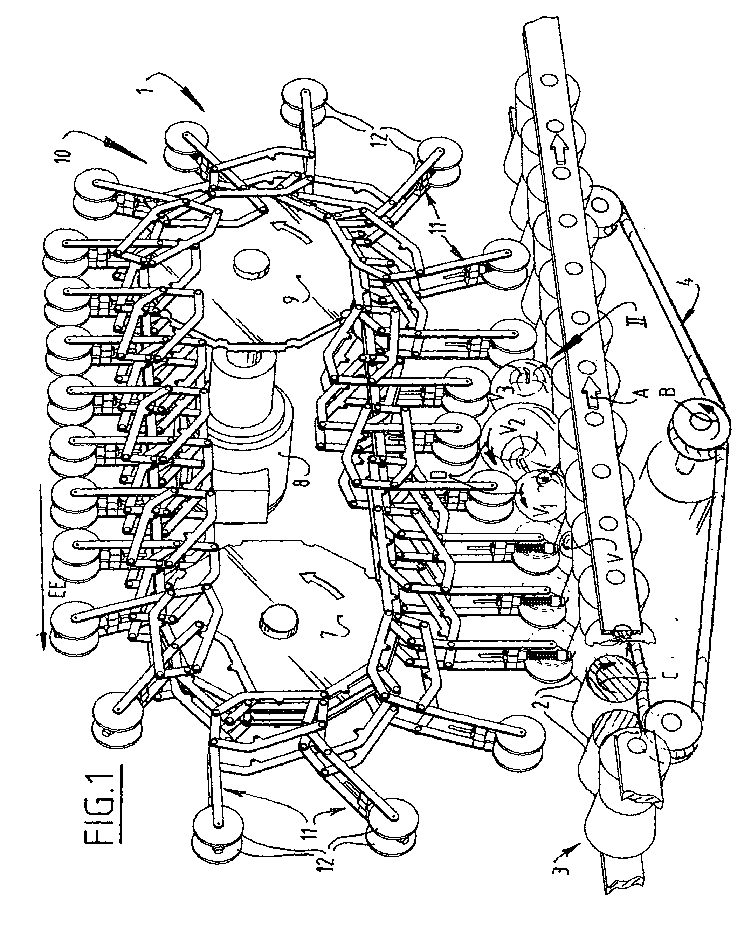

In a sorting line 1 (figure 1) fruits V₁, V₂ and V₃, for instance of differing size, are supplied in the direction of arrow A on a conveyor 3 provided with diabolos 2. Using a belt, rope or chain 4 driven in the direction of arrow B, the diabolos are rotated in a direction according to arrow C, whereby the fruits rotate in the direction of arrow D. The rotation direction D of the fruits, which is in fact in the direction opposite to transporting direction A, is recommended since, as will become apparent hereinbelow, the hardness of the fruits is determined by means of mechanical contact. The advantage of this transporting direction is that the fruit displaces little during the momentary mechanical contact, less damage to the fruits will occur and/or the influence of occurring lateral forces will be less in evidence.

The above stated advantage is particularly important in a stationary disposition (not further shown), wherein a number of contact elements is disposed successively above the conveyor with diabolos. In the embodiment shown in figure 1, wherein the contact elements co-displace, it is also possible to envisage the fruits being rotated forward.

Arranged above conveyor 3 is a device 10 which comprises a number of contact elements 11 which are connected mutually pivotally and which are also provided on the ends with one or more discs or wheels 12 to allow such an element 11 to roll over a fruit V₁-V₃.

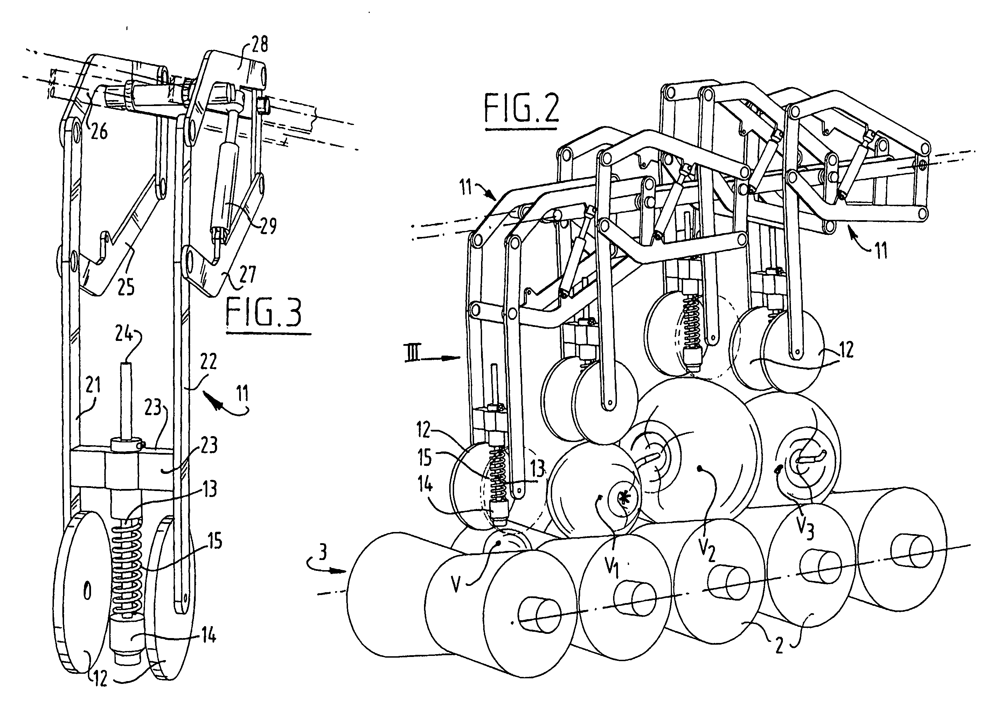

Reversing wheels 7 respectively 9 are driven using a schematically designated motor 8 in order to drive the contact elements 11 in the direction of arrow E at substantially the same speed as the transporting speed of conveyor 3, i.e. synchronously therewith. As shown particularly clearly in figures 2 and 3, a pin 13 with a weight member 14 of predetermined value, for instance 10 grammes, is situated in each case adjacently of a disc or wheel 12, wherein the weight is held in the rest position by a helical pressure spring 15. While a contact element-11 co-displaces with a determined fruit, a solenoid (not further shown) is released at predetermined times, whereby the weight 14 of predetermined mass on the impactor element drops against the surface of a fruit V₁, V₂ or V₃, whereby the degree of impact of that weight against the fruit, and thus the hardness of this fruit, can be determined by a piezo-electric transducer (not shown). An example of the co-action of a solenoid and piezo-electric element is described in for instance the above stated international patent application.

Because the contact elements co-displace with the conveyor and the fruit is simultaneously rotated, the hardness of the fruit can for instance be measured at 10 or more locations along the whole periphery of this fruit. In the case of small fruits a smaller number of measurements may already suffice.

As shown particularly in figure 3, contact element 11 comprises two parallel arms 21, 22 with a connecting piece 23 arranged therebetween, wherein the solenoid and the piezo-electric element are arranged in a manner not shown. An electric lead 24 is also connected to this connecting piece 23. Pairs of curved arms 25, 26 respectively 27, 28, together forming a parallelogram, are further arranged pivotally on the mounting arm, while in the present embodiment a damper 29 (and/or a spring) is arranged between arms 27 and 28 in order to prevent damage to the fruits and/or optimize the response of the impactor. A subsequent contact element 11 can be mounted pivotally on contact element 11, as elucidated in figures 1 and 2, so that the successive contact elements 11 can follow the fruits of differing diameter as well as possible.

In another, not shown, preferred embodiment the contact elements can likewise be mounted directly on a chain or toothed belt, wherein it must however be ensured that the contact elements can move sufficiently in vertical direction.

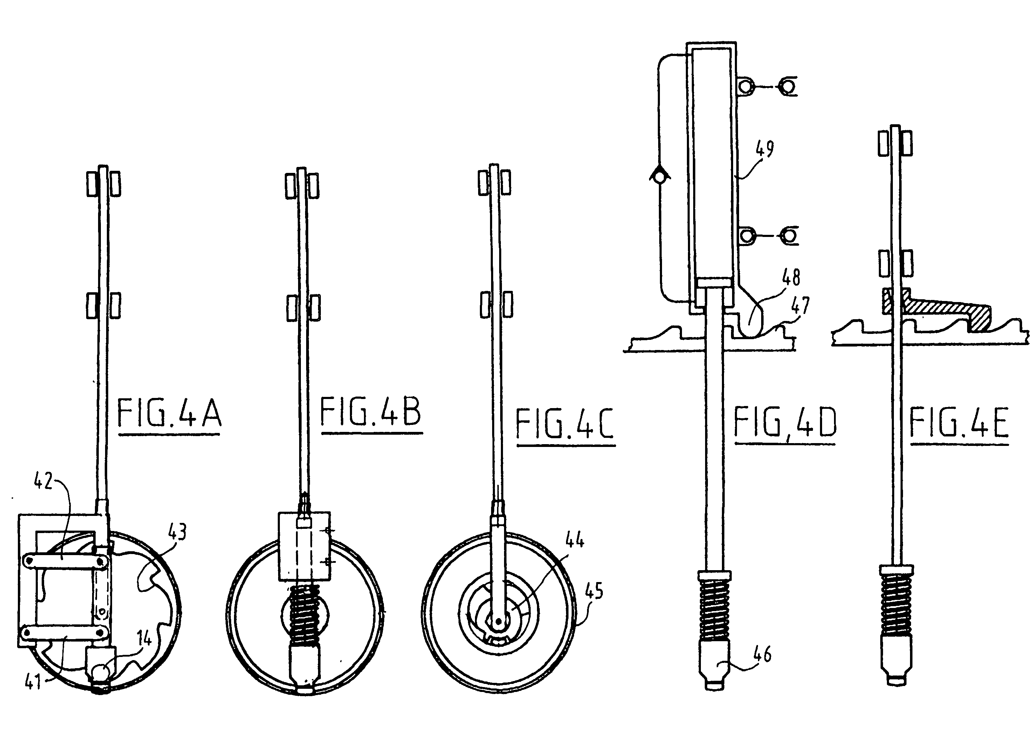

In the embodiments according to figures 4A, 4C, 4D and 4E are shown schematic alternatives to the embodiment according to figure 4B. In figure 4A a weight 14 is moved mechanically upward in each case using parallel arms 41, 42 and a curve disc 43, while this mechanical transport is realized according to figure 4C using a curve 44 arranged internally on a disc 45, whereby the mass with the piezo-element is raised, whereafter it falls freely onto the inner side of the wheel. The pulse of the impact transmitted by the wheel to the measured product is measured by the piezo-element.

In the embodiment according to figure 4D a weight 46 is moved upward using a tooth path 47 and a cam 48, whereafter it can fall, while in order to avoid damage to the fruits a damper 49 can also be arranged. In the embodiment of figure 4E the damping can be obtained in a manner corresponding to the embodiment shown in figures 1-3.

The preferred embodiments applied in figures 4A-4E can likewise be applied in a stationary arrangement, i.e. wherein a number of such impactor elements is disposed mutually adjacently above the conveyor provided with diabolos, wherein the fruits are rotated and are measured in each case at a further location on the periphery thereof under a following impactor element.

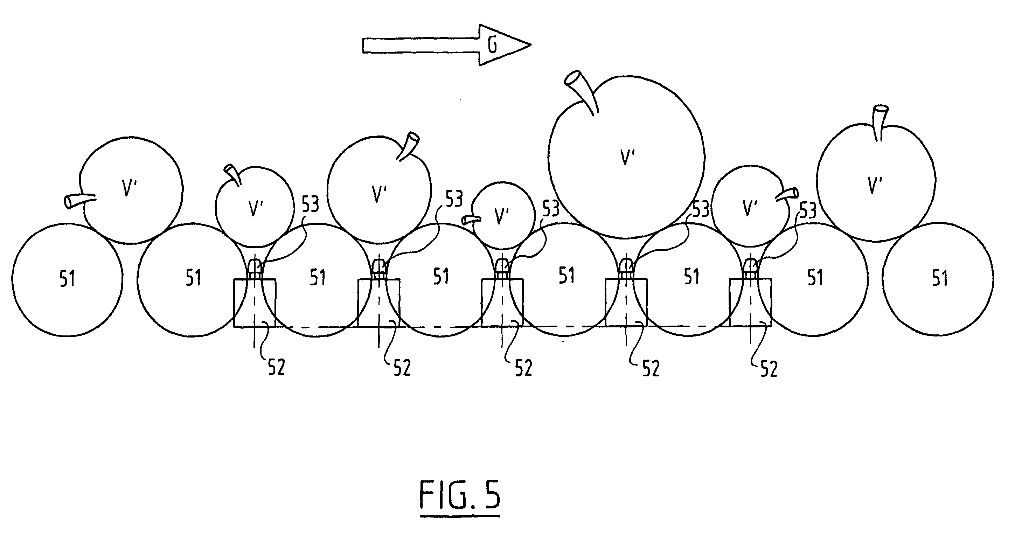

In the preferred embodiment shown in figure 5 the fruits V' of different diameter are transported in the direction of arrow G on a conveyor provided with diabolos 51, wherein impactor element 52 co-displaces between the diabolos 51 and during rotation of fruits V' a contact element 53 is jolted upward each time against fruits V' with a predetermined impulse, wherein in the manner described above the impact thereof is measured with for instance a piezo-electric element.

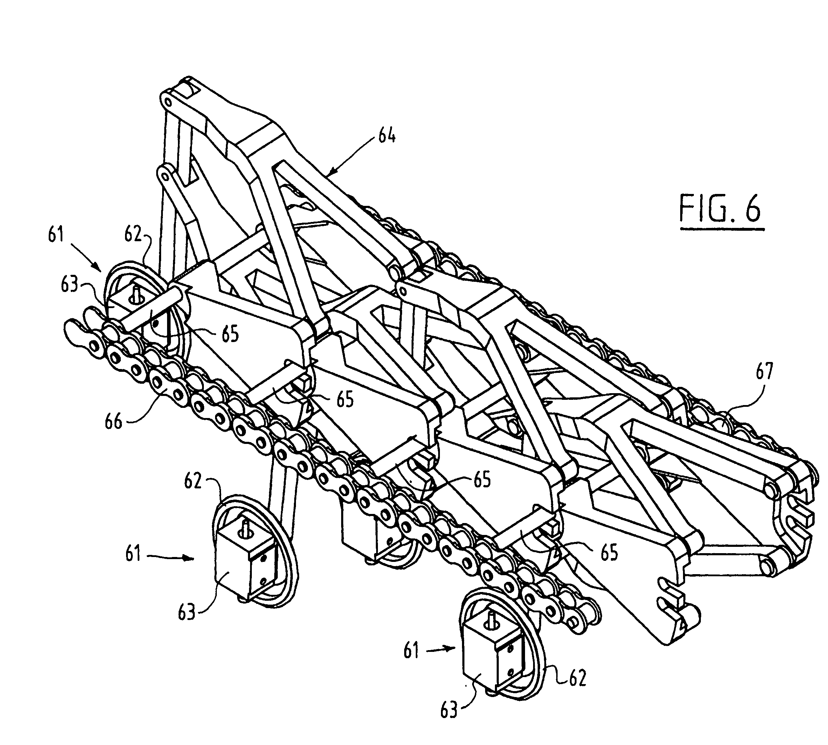

In the embodiment shown in figure 6, which forms a further development of the embodiment shown in figures 1, 2 and 3, each contact element 61 is provided with a single disc 62, within the periphery of which the impactor members 63 are arranged in each case. The construction 64, which enables elements 61 to move as freely as possible in vertical direction, is arranged on pins 65 which are advanced between chains 66 and 67 synchronously with the conveyor with diabolos, not shown in figure 6.

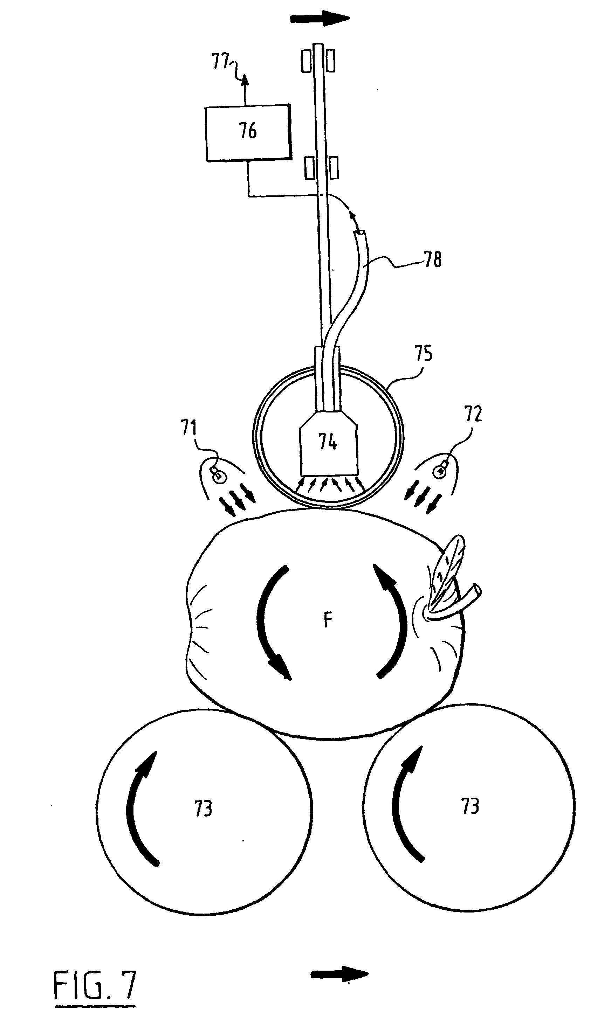

In the embodiment according to figure 7 light is projected onto a fruit F rotated by transport rollers 73 using two light sources 71, 72, the reflection of which light is determined using an optical transducer 74 arranged adjacently of a wheel 75 which follows the surface of fruit F. Connected to transducer 74 is a glass fibre cable 78 which is connected on the other side to an analysis device 76 for analysing the spectrum of the reflected light, on the basis of which an output signal 77 is generated by analysis device 76, which signal is a measure for the colour of the fruit.

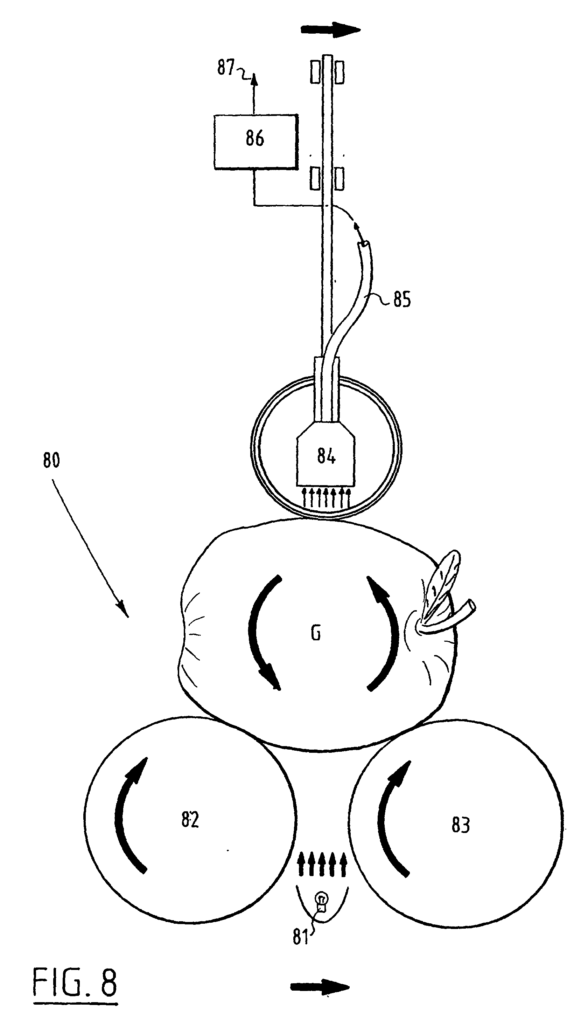

In the device 80 according to figure 8 light is projected between two transport rollers 82, 83 onto a fruit G by a light source 81, whereby fruit G is rotated, while the quantity of light let through by fruit G is measured by a transducer 84 which is connected to a spectral analyser 86 via a glass fibre cable 85, the output signal 87 of which analyser provides a measure for the quantity of light let through by the object.

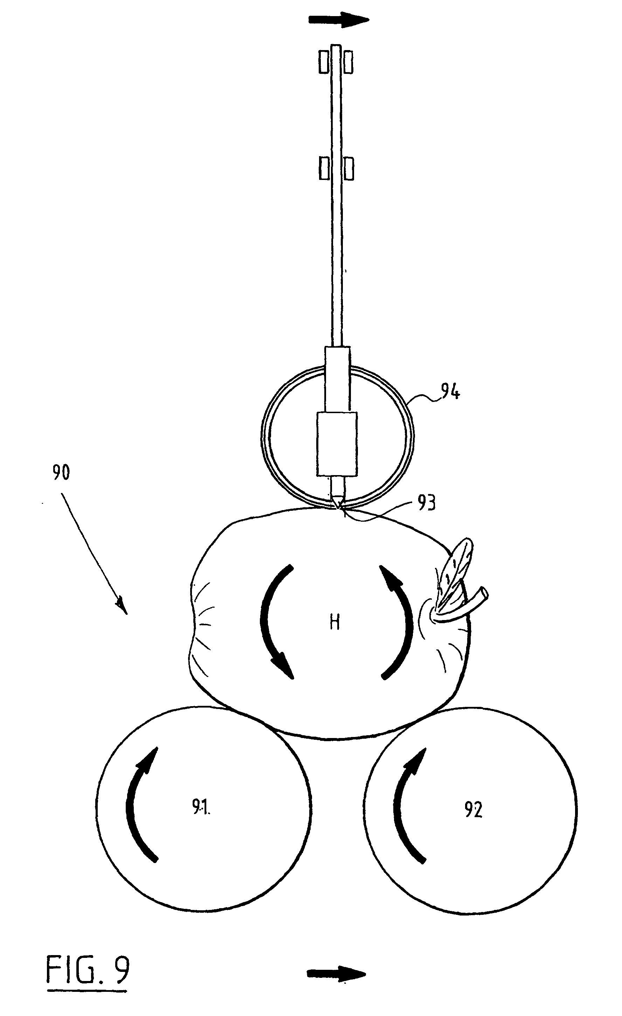

In the device 90 according to figure 9 a fruit H set into rotation by transport rollers 91 and 92 is scanned using a scanning member 93, which is arranged adjacently of a tracking wheel 94 in order to enable determining of the roughness of the surface of fruit H from the movements of scanning member 93.

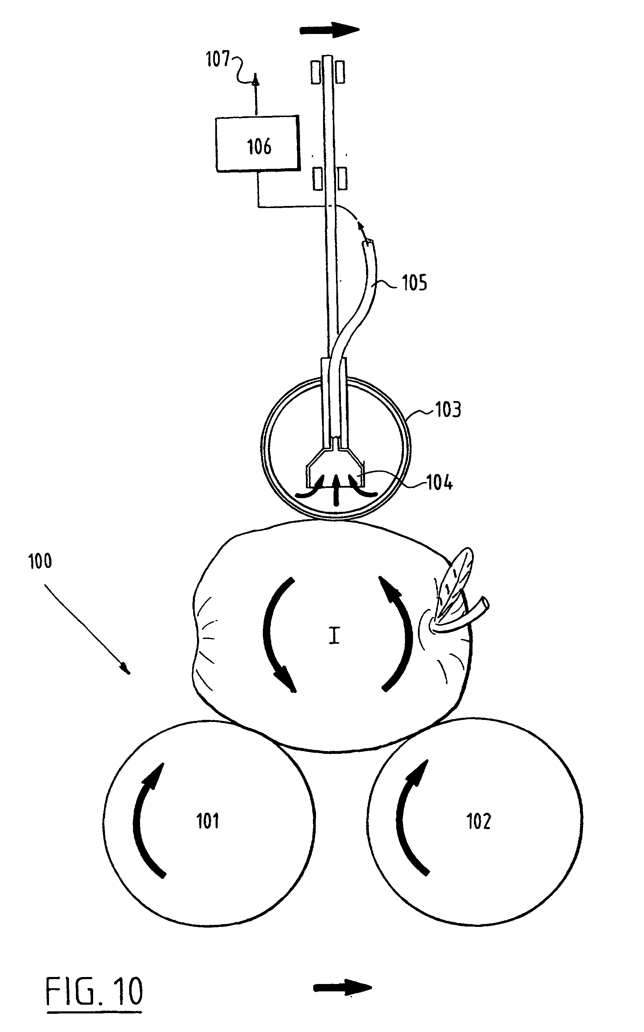

In the device 100 of figure 10, wherein a fruit I is rotated by transport rollers 101, 102, the gases given off by fruit I are taken up using a sniffer member 104 arranged adjacently of a tracking wheel 103 and guided further via a hose 105 to a gas analysis device 106 for the purpose of generating an electrical signal 107 which forms a measure for the quantity and/or composition of the gases given off by a fruit, and thus for instance for the ripeness thereof.

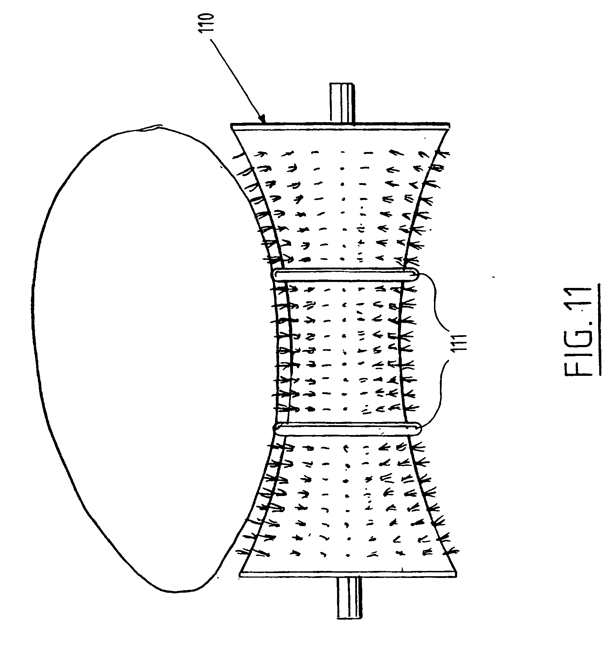

In the case of for instance elongate and vulnerable fruits, such as avocados and pears, which are usually supplied on brush rollers, the problem can occur of these fruits being urged sideward when there is contact with a wheel on the upper side. In order to prevent this the brush roller 110 of figure 11 is provided with two rubber rings 111, which ensure that a fruit undergoes sufficient lateral resistance.

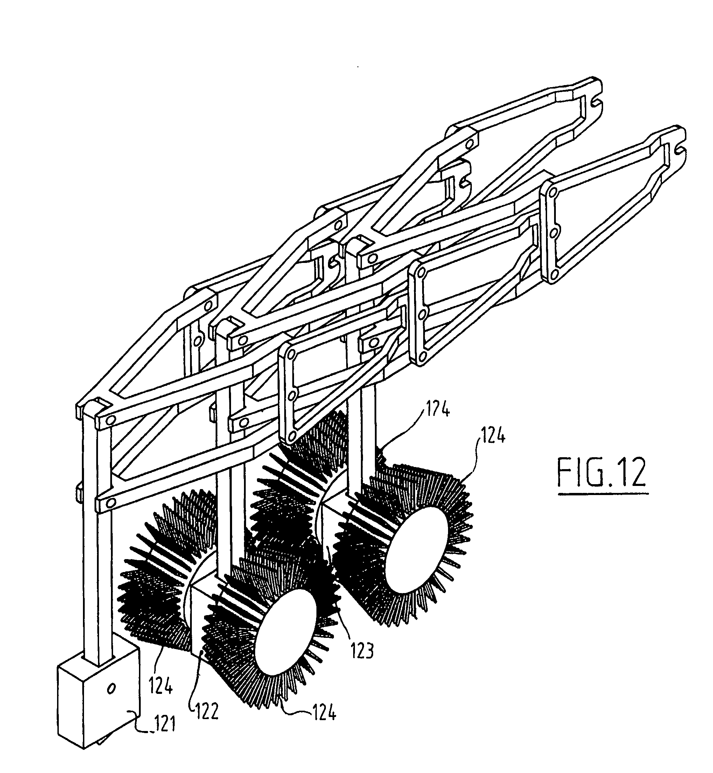

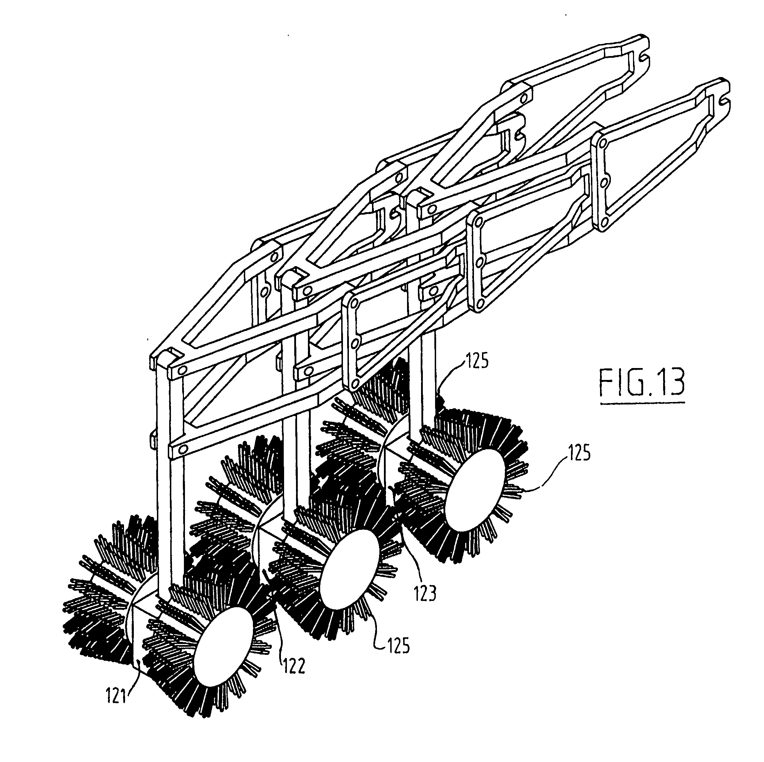

In a further preferred embodiment according to figures 12 and 13 sensor elements 121, 122 and 123 are arranged between diabolo-shaped brushes 124 and 125, the density of which is different. The diabolo-shaped brushes enable good positioning of the fruits and an accurate and reliable measuring result can be obtained.

Dependent on the type of fruits, the rotating elements can however also be a diabolo-shaped smooth surface or even be provided with a flat surface of for instance plastic or rubber, which can also be locally rough.

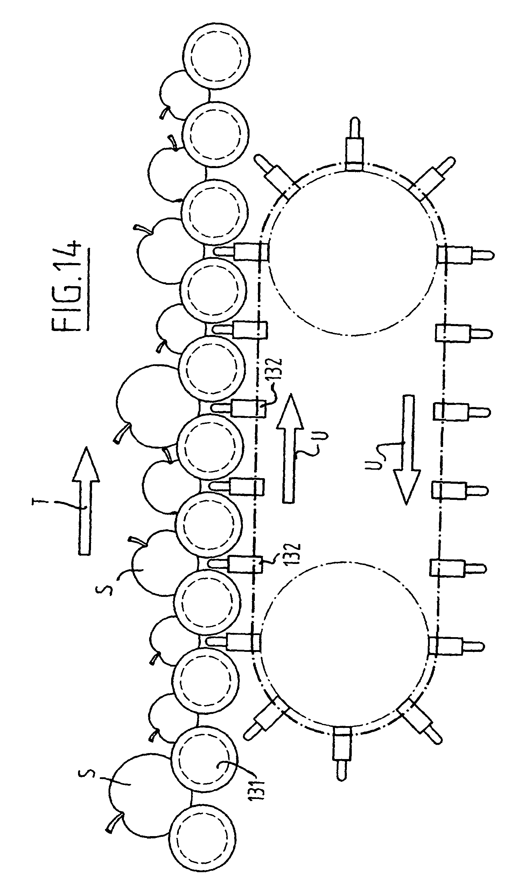

In the embodiment of fig. 14 the apples are supplied on diabolos 131 in the direction of arrow T. Sensors 132 are arranged on an endless belt 133 in the direction of arrows U which is moved synchronously with diabolos 131 such that the sensors protrude in each case precisely therebetween. This embodiment is particularly advantageous with batches of fruits with very large difference in dimensions.

## Claims

1. Apparatus for determining a property of a vegetable or fruit, comprising:
a supply conveyor (3) for supplying the vegetables or fruits, the vegetables or fruits being rotated on the supply conveyor; and
one or more sensor members for measuring the property of the vegetables or fruits during rotation thereof wherein during rotation of the fruit the sensor member is brought a number of times into the vicinity of the fruit or the vegetable, wherein the property is measured, in order to determine the property of a vegetable or fruit over at least a portion of the surface thereof, **characterised in that**
- a number of firmness measuring members are arranged successively and are coupled to drive means for driving thereof at substantially the same speed as the supply conveyor (3);
- each individual sensor member co-displaces with an individual vegetable or fruit at more or less the same speed; and
- each individual sensor member performs one or more measurements on one individual fruit or vegetable.

2. Apparatus as claimed in claim 1, wherein the sensor members are arranged adjacently of one or more wheels (12).

3. Apparatus as claimed in claim 1 or 2, wherein the supply conveyor (3) is provided with brush rollers with slip-resistant means.

4. Apparatus according to claim 1, 2 or 3 for determining the firmness of a vegetable or fruit, wherein the sensor members are arranged above the supply conveyor to enable a more or less perpendicular measurement on the fruits or vegetables.

5. Apparatus as claimed in claim 4, wherein successive firmness measuring members are arranged pivotally relative to each other.

6. Apparatus as claimed in any of the claims 1-5, wherein a firmness measuring member comprises an (endless) tooth path (47) or curve discs (43).

7. Method for determining the firmness of a vegetable or fruit, consisting of the steps:
rotating the vegetable or fruit;
carrying an impactor element close to the surface of a fruit or vegetable while being co-displaced with the fruits or vegetables during rotation of the fruit;
bringing an individual impactor element into contact with an individual fruit or vegetable a number of times; and
measuring each time the impact of the respective impactor element in order to determine the firmness of the vegetable or fruit over at least a portion of the surface thereof.

8. Method as claimed in claim 7, wherein the impact is determined using a (piezo-electric) transducer that measures a number of times on different surfaces of the same fruit or vegetable.

9. Method as claimed in claim 7 or 8, wherein the fruit or vegetable is transported over a conveyor (3) and wherein the impactor element co-displaces with the fruit and measures more or less perpendicular to the surface area of the fruits or vegetables.

## Patentansprüche

1. Vorrichtung zum Bestimmen einer Eigenschaft eines Stücks Gemüse oder einer Frucht, umfassend:
einen Zufuhrförderer (3) zum Zuführen von Gemüse oder Früchten, wobei das Gemüse oder die Früchte auf dem Zufuhrförderer gedreht werden, und
ein oder mehrere Sensorelemente zum Messen der Eigenschaft des Gemüses oder der Früchte während deren Drehung, wobei während der Drehung der Frucht das Sensorelement mehrere Male in die Nähe der Frucht oder des Gemüsestücks gebracht wird, wobei die Eigenschaft gemessen wird, um die Eigenschaft eines Stücks Gemüse oder einer Frucht über mindestens einem Teil der Oberfläche davon zu bestimmen, **dadurch gekennzeichnet, dass**
- eine Reihe von Festigkeitsmesselementen aufeinanderfolgend angeordnet sind und mit Antriebseinrichtungen gekoppelt sind zum Bewegen derselben bei im wesentlichen derselben Geschwindigkeit wie dem Zufuhrförderer (3);
- jedes einzelne Sensorelement zusammen mit dem einzelnen Stück Gemüse oder der einzelnen Frucht bei mehr oder weniger derselben Geschwindigkeit versetzt wird; und
- jedes einzelne Sensorelement eine oder mehrere Messung(en) bei einer einzelnen Frucht oder einem einzelnen Stück Gemüse ausführt.

2. Vorrichtung wie im Anspruch 1 beansprucht, wobei die Sensorelemente neben einem oder mehreren Rädern (12) angeordnet sind.

3. Vorrichtung wie im Anspruch 1 oder 2 beansprucht, wobei der Zufuhrförderer (3) über rutschresistente Einrichtungen mit Börstenrollen ausgestattet ist.

4. Vorrichtung gemäß Anspruch 1, 2 oder 3 zum Bestimmen der Festigkeit eines Stücks Gemüse oder einer Frucht, wobei die Sensorelemente oberhalb des Zufuhrförderers angeordnet sind, um eine mehr oder weniger senkrechte Messung gegenüber den Früchten oder dem Gemüse zu ermöglichen.

5. Vorrichtung wie im Anspruch 4 beansprucht, wobei aufeinanderfolgende Festigkeitsmesselemente zueinander schwenkbar angeordnet sind.

6. Vorrichtung wie in irgendeinem der Ansprüche 1 bis 5 beansprucht, wobei ein Festigkeitsmesselement eine (endlose) Zahnführung (47) oder gekrümmte Scheiben (43) aufweist.

7. Verfahren zum Bestimmen der Festigkeit eines Stücks Gemüse oder einer Frucht, bestehend aus den Schritten:
Drehen des Stücks Gemüse oder der Frucht;
Bringen eines Aufprallelements in die Nähe der Oberfläche einer Frucht oder eines Stücks Gemüse, während es zusammen mit den Früchten oder dem Gemüse beim Drehen der Frucht versetzt wird;
in Kontakt bringen eines einzelnen Aufprallelements mehrere Male mit einer einzelnen Frucht oder einem einzelnen Stück Gemüse; und
Messen bei jedem Aufprall des jeweiligen Aufprallelements, um die Festigkeit des Stücks Gemüse oder der Frucht an mindestens einem Teil der Oberfläche davon zu bestimmen.

8. Verfahren wie im Anspruch 7 beansprucht, wobei der Aufprall bestimmt wird unter Verwendung eines (piezo-elektrischen) Umwandlers, der mehrere Male auf verschiedenen Oberflächenteilen derselben Frucht oder desselben Stücks Gemüse misst.

9. Verfahren wie im Anspruch 7 oder 8 beansprucht, wobei die Frucht oder das Stück Gemüse über einen Förderer (3) transportiert wird, und wobei das Aufprallelement zusammen mit der Frucht versetzt wird und mehr oder weniger senkrecht zur Oberfläche der Früchte oder des Gemüses misst.

## Revendications

1. Dispositif de détermination d'une propriété d'un légume ou d'un fruit, comprenant :
un convoyeur d'alimentation (3) destiné à amener les légumes ou les fruits, les légumes ou les fruits étant mis en rotation sur le convoyeur d'alimentation ; et
un ou plusieurs éléments formant capteur destinés à mesurer la propriété des légumes ou des fruits pendant la rotation de ceux-ci, dans lequel pendant la rotation du fruit, l'élément formant capteur est amené un certain nombre de fois à proximité du fruit ou du légume, dans lequel la propriété est mesurée, pour déterminer la propriété d'un légume ou d'un fruit sur au moins une partie de la surface de celui-ci,
**caractérisé en ce que**
un nombre d'éléments de mesure de fermeté est agencé successivement et est couplé à des moyens d'entraînement pour entraîner ceux-ci sensiblement à la même vitesse que le convoyeur d'alimentation (3) ;
chaque élément formant capteur individuel se déplace conjointement avec un légume ou un fruit individuel plus ou moins à la même vitesse ; et
chaque élément formant capteur individuel réalise une ou plusieurs mesures sur un fruit ou un légume individuel.

2. Dispositif selon la revendication 1, dans lequel les éléments formant capteur sont agencés de manière adjacente à une ou plusieurs roues (12).

3. Dispositif selon la revendication 1 ou 2, dans lequel le convoyeur d'alimentation (3) est pourvu de rouleaux à brosse avec des moyens résistants au dérapage.

4. Dispositif selon la revendication 1, 2 ou 3, destiné à déterminer la fermeté d'un légume ou d'un fruit, dans lequel les éléments formant capteur sont agencés au-dessus du convoyeur d'alimentation pour permettre une mesure plus ou moins perpendiculaire sur les fruits ou les légumes.

5. Dispositif selon la revendication 4, dans lequel des éléments de mesure de fermeté successifs sont agencés en pivotement l'un par rapport à l'autre.

6. Dispositif selon l'une quelconque des revendications 1 à 5, dans lequel un élément de mesure de fermeté comprend une trajectoire dentée (sans fin) (47) ou des disques incurvés (43).

7. Procédé de détermination de la fermeté d'un légume ou d'un fruit, comprenant les étapes consistant à :
mettre le légume ou le fruit en rotation ;
porter un élément impacteur proche de la surface d'un fruit ou d'un légume tout en étant déplacé conjointement avec les fruits ou les légumes pendant la rotation du fruit ;
amener un élément impacteur individuel en contact avec un fruit ou un légume individuel un certain nombre de fois ; et
mesurer chaque fois l'impact de l'élément impacteur respectif pour déterminer la fermeté du légume ou du fruit sur au moins une partie de la surface de celui-ci.

8. Procédé selon la revendication 7, dans lequel l'impact est déterminé à l'aide d'un transducteur (piézoélectrique) qui mesure un certain nombre de fois sur différentes surfaces du même fruit ou légume.

9. Procédé selon la revendication 7 ou 8, dans lequel le fruit ou le légume est transporté sur un convoyeur (3) et dans lequel l'élément impacteur se déplace conjointement avec le fruit et mesure de façon plus ou moins perpendiculaire à la zone de surface des fruits ou des légumes.
